# EUROPÄISCHE PATENTSCHRIFT

(11) **EP 3 441 004 B1**
(45) Veröffentlichungstag und Bekanntmachung des Hinweises auf die Patenterteilung: **11.12.2019**
(21) Anmeldenummer: 17185953.1
(22) Anmeldetag: 11.08.2017
(51) Int. Cl.: A61B 6/00, A61B 6/03

(54) **VERFAHREN ZUR AUFNAHME EINES BILDDATENSATZES MIT EINEM RÖNTGENBILDGEBUNGSSYSTEM UND RÖNTGENBILDGEBUNGSSYSTEM**
METHOD FOR RECORDING AN IMAGE DATASET WITH AN X-RAY IMAGING SYSTEM AND X-RAY IMAGING SYSTEM
PROCÉDÉ DE RÉCEPTION D'UN ENSEMBLE DE DONNÉES D'IMAGE AU MOYEN D'UN SYSTÈME D'IMAGERIE À RAYONS X ET SYSTÈME D'IMAGERIE À RAYONS X

(43) Veröffentlichungstag der Anmeldung: 13.02.2019
(73) Patentinhaber: Siemens Healthcare GmbH, 91052 Erlangen (DE)
(72) Erfinder: Hornung, Oliver, 91077 Dormitz (DE); Kowarschik, Markus, 90408 Nürnberg (DE)

(56) Entgegenhaltungen:
- EP-A1- 2 633 817
- DE-A1-102009 061 749
- US-A1- 2016 239 971

## Beschreibung

Die Erfindung betrifft ein Verfahren zur Aufnahme eines Bilddatensatzes mit einem Röntgenbildgebungssystem gemäß dem Patentanspruch 1 sowie ein Röntgenbildgebungssystem zur Durchführung eines derartigen Verfahrens gemäß dem Patentanspruch 7.

In der 3D- und 4D-Röntgenbildgebung werden im Allgemeinen Röntgenbildgebungssysteme verwendet, bei denen an einer Halterung (z.B. ein C-Bogen) ein Aufnahmesystem mit einem Röntgendetektor und einer Röntgenquelle angeordnet ist und um ein Untersuchungsobjekt rotiert, so dass Projektionsbilder aus einer Vielzahl von Projektionsrichtungen aufgenommen und rekonstruiert werden können. Ein grundsätzliches technisches Problem bei einem solchen Aufbau besteht darin, dass zur Kalibrierung von 3D und 4D Akquisitionsprotokollen ein sehr hoher Zeitaufwand, z.B. mehrere Tage, benötigt wird. Ein Röntgenbildgebungssystem mit einer Kalibrierung ist z.B. aus der US 2016/0239971 bekannt. Es zeigt die üblichen Merkmale einer Röntgenquelle und eines Röntgendetektors mit einer Systemsteuerung und einer Eingabe/Ausgabe-Einheit.

Insbesondere werden angiographische 3D-Aufnahmen durch die mechanischen Eigenschaften der Halterung eingeschränkt. Diese Einschränkung hat unter anderem zur Folge, dass jedes 3D Akquisitionsprotokoll separat vorab kalibriert werden muss. Derartige Protokolle unterscheiden sich bspw. in der Anzahl der Projektionsbilder, dem abzutastenden Winkelbereich und der Akquisitionszeit. Dies bedingt offensichtlich einen hohen Aufwand (Kombinatorik), d.h. für jede Parameteränderung wird i.A. ein neues Akquisitionsprotokoll benötigt. Als konkrete Einschränkung heutiger bekannter Systeme ergibt sich z.B., dass die Vorgabe der Akquisitionszeit unmittelbar während einer Intervention nicht möglich ist wegen zu hoher resultierender Kalibrieraufwände. Jedoch ist ein solches Feature wünschenswert, z.B. im Hinblick auf Verfahren wie die 4D DSA (Zeitaufgelöste dreidimensionale Digiale Subtraktionsangiographie), bei welcher sich die Akquisitionsdauer idealerweise aus physiologischen Gegebenheiten ergibt (Hämodynamik).

Es ist Aufgabe der vorliegenden Erfindung, ein Verfahren zur Aufnahme eines Bilddatensatzes mit einem Röntgenbildgebungssystem bereitzustellen, welches die Nacheile des Standes der Technik reduziert; des Weiteren ist es Aufgabe der Erfindung, ein für die Durchführung des Verfahrens geeignetes Röntgengerät bereitzustellen.

Die Aufgabe wird erfindungsgemäß gelöst durch ein Verfahren zur Aufnahme eines Bilddatensatzes mit einem Röntgenbildgebungssystem gemäß dem Patentanspruch 1 und von einem Röntgenbildgebungssystem gemäß dem Patentanspruch 7. Vorteilhafte Ausgestaltungen der Erfindung sind jeweils Gegenstand der zugehörigen Unteransprüche.

Durch das erfindungsgemäße Verfahren zur Akquisition eines Bilddatensatzes mit einem Röntgenbildgebungssystem mit einem um ein Untersuchungsobjekt rotierbaren Aufnahmesystem, welches für eine Endlosrotation (also eine unbegrenzte Rotationsfähigkeit in beide Richtungen) ausgebildet ist, mit den Schritten: Bereitstellung eines Kalibrierdatensatzes, welcher Messdaten aus einer Vielzahl von Rotationen des Aufnahmesystems in Endlosrotation aufweist, Entgegennahme zumindest eines auswählbaren Akquisitionsparameters insbesondere aus der Gruppe: Dauer der Akquisition, Anzahl der zu akquirierenden Projektionsbilder, aufzunehmender Winkelbereich der Akquisition und Winkelinkrement zwischen je zwei aufeinander abfolgenden Projektionsbildern, Ermittlung, insbesondere Berechnung, eines Akquisitionsprotokolls mit dem oder den ausgewählten Akquisitionsparametern aus dem bereitgestellten Kalibrierdatensatz, Laden des ermittelten Akquisitionsprotokolls, und Aufnahme eines Bilddatensatzes unter Verwendung des ermittelten Akquisitionsprotokolls, wird auf einfache Weise eine Möglichkeit geschaffen, Akquisitionsprotokolle aus bereits vorhandenen Kalibrierdaten zu berechnen, ohne bei Änderung der Akquisitionsparameter jedes einzelne Mal eine neue Kalibration durchführen zu müssen. Die Voraussetzung dafür bilden neuartige, besonders stabil rotierende Aufnahmesysteme (z.B. C-Bögen), die mit gleichbleibender Dynamik pro Rotationsgeschwindigkeit rotieren können. Durch das erfindungsgemäße Verfahren können Zeit und Aufwand für Neukalibrierungen eingespart werden und es können flexibel auch während Interventionen und anderen zeitkritischen Anwendungen 3D und 4D-Aufnahmen mit frei wählbaren Akquisitionsparametern durchgeführt werden. Der Kalibrierdatensatz, der bereitgestellt wird, enthält dabei idealerweise Messdaten von möglichst vielen bzw. allen möglichen Rotationsgeschwindigkeiten inklusive des sogenannten Subsamplings.

Nach einer weiteren Ausführung der Erfindung wird zumindest ein weiterer Akquisitionsparameter aus der Gruppe Dauer der Akquisition, Anzahl der zu akquirierenden Projektionsbilder, aufzunehmender Winkelbereich der Akquisition und Winkelinkrement zwischen je zwei aufeinander abfolgenden Projektionsbildern entgegengenommen, ein weiteres Akquisitionsprotokoll berechnet, das weitere Akquisitionsprotokoll geladen und daraus ein weiterer Bilddatensatz aufgenommen. Dies kann direkt nach der Aufnahme des ersten Bilddatensatzes durchgeführt werden, z.B. wenn während eines interventionellen Eingriffs eine Änderung eines Akquisitionsparameters benötigt wird.

Nach einer weiteren Ausführung der Erfindung wird z.B. mittels einer Anzeigeeinheit an einen Nutzer des Röntgenbildgebungssystems eine Abfrage bezüglich des auswählbaren Akquisitionsparameters aus der Gruppe: Dauer der Akquisition, Anzahl der zu akquirierenden Projektionsbilder, aufzunehmender Winkelbereich der Akquisition und Winkelinkrement zwischen je zwei aufeinander abfolgenden Projektionsbildern, durchgeführt. In diesem Fall wird der Nutzer direkt zu gewünschten Akquisitionsparametern befragt und kann diese anschließend z.B. mittels einer Eingabeeinheit eingeben, so dass sie dann von dem Röntgenbildgebungssystem entgegengenommen werden können. Alternativ können die Akquisitionsparameter auch automatisch z.B. aufgrund von Anforderungen des Organprogrammes oder anderer Voraussetzungen ausgewählt und entgegen genommen werden. Wichtig ist, dass eine flexible Auswahl der Akquisitionsparameter möglich ist.

Alternativ zu den genannten können auch weitere über die Gruppe Dauer der Akquisition, Anzahl der zu akquirierenden Projektionsbilder, aufzunehmender Winkelbereich der Akquisition und Winkelinkrement zwischen je zwei aufeinander abfolgenden Projektionsbildern hinausgehende Akquisitionsparameter entgegengenommen und verwendet werden.

Zur Durchführung des Verfahrens ist erfindungsgemäß ein Röntgenbildgebungssystem mit einem Aufnahmesystem mit einem Röntgendetektor und einer Röntgenquelle, die an einer Halterung angeordnet sind, welche Halterung dazu ausgebildet ist, in einer Endlosrotation (also eine hinsichtlich des Drehwinkels uneingeschränkte Rotierbarkeit) um ein Untersuchungsobjekt zu rotieren, vorgesehen, wobei das Röntgenbildgebungssystem dazu ausgebildet ist, während der Rotation des Aufnahmesystems eine Vielzahl von Projektionsbildern aus unterschiedlichen Projektionsrichtungen sowie einen Bilddatensatz gemäß einem Akquisitionsprotokoll aufzunehmen, aufweisend eine Systemsteuerung, welche dazu ausgebildet ist, einen zuvor aufgenommenen Kalibrierdatensatz, welcher Messdaten aus einer Vielzahl von Rotationen des Aufnahmesystems in Endlosrotation aufweist, bereitzustellen und ein Akquisitionsprotokoll zu laden, aufweisend eine Eingabeeinheit, welche zur Entgegennahme zumindest eines auswählbaren Akquisitionsparameters aus der Gruppe Dauer der Akquisition, Anzahl der zu akquirierenden Projektionsbilder, aufzunehmender Winkelbereich der Akquisition und Winkelinkrement zwischen je zwei aufeinander abfolgenden Projektionsbildern, ausgebildet ist und aufweisend eine Recheneinheit, welche dazu ausgebildet ist, ein Akquisitionsprotokoll mit dem oder den ausgewählten Akquisitionsparametern aus dem bereitgestellten Kalibrierdatensatz zu berechnen.

Nach einer Ausführung der Erfindung wird die Halterung von einem C-Bogen gebildet und ist der C-Bogen mittels einer Schleifringkonstruktion aufgehängt ist, so dass eine Endlosrotation des C-Bogens ausführbar ist.

Zweckmäßigerweise weist das Röntgenbildgebungssystem eine Anzeigeeinheit zur Anzeige einer Abfrage bezüglich des oder der Akquisitionsparameter und eine Eingabeeinheit zur Eingabe der entsprechenden Akquisitionsparameter auf.

Die Erfindung sowie weitere vorteilhafte Ausgestaltungen gemäß Merkmalen der Unteransprüche werden im Folgenden anhand schematisch dargestellter Ausführungsbeispiele in der Zeichnung näher erläutert, ohne dass dadurch eine Beschränkung der Erfindung auf diese Ausführungsbeispiele erfolgt. Es zeigen:
- FIG 1: eine Ansicht eines erfindungsgemäßen Röntgenbildgebungssystems; und

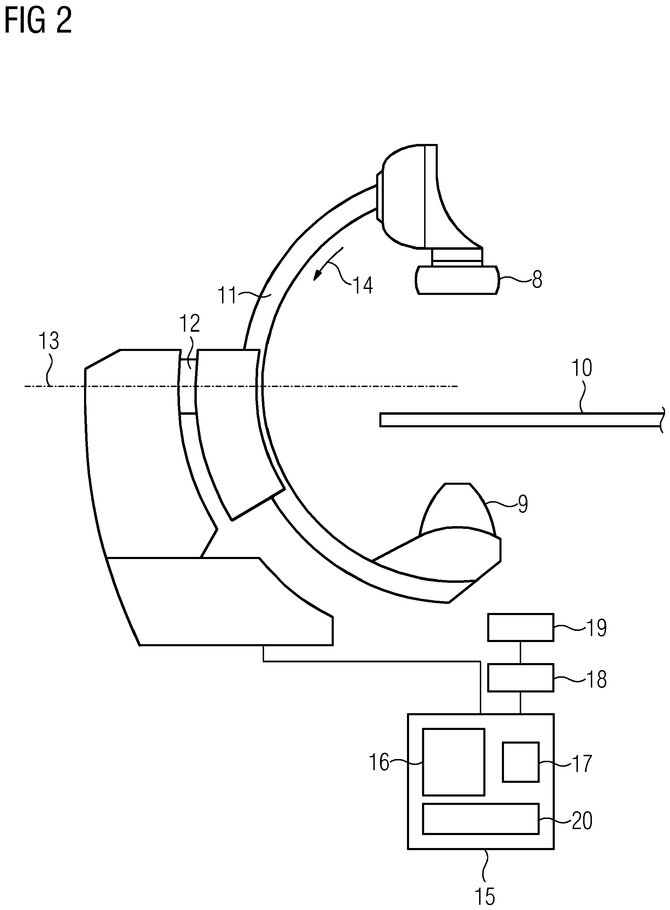

- FIG 2: ein Ablauf eines erfindungsgemäßen Verfahrens zur Akquisition eines Bilddatensatzes.

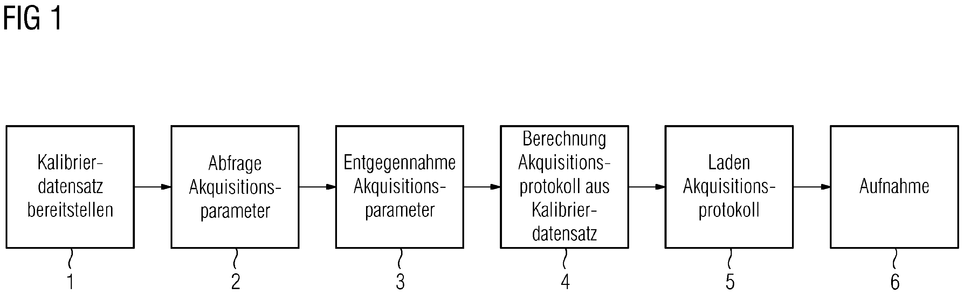

In der FIG 1 ist ein beispielhaftes Röntgenbildgebungssystem mit einem C-Bogen 11 gezeigt, welches zur Durchführung des Verfahrens geeignet ist. An dem C-Bogen 11 sind an einem Ende eine Röntgenquelle 9 und an einem anderen Ende ein Röntgendetektor 8 angeordnet. Der C-Bogen 11 kann in mehreren Ebenen bewegt werden, unter anderem kann er in Richtung des Pfeils 14 verschoben werden und um die Achse 13 rotiert werden. Bei dieser Rotation um ein auf dem Patiententisch 10 angeordnetes Untersuchungsobjekt kann eine Vielzahl von Projektionsbildern des Untersuchungsobjekts aus unterschiedlichen Projektionsrichtungen (Angulationen) aufgenommen werden. In der Aufhängung 12 ist beispielhaft eine Schleifringkonstruktion angeordnet, durch die der C-Bogen 11 in einer Endlosrotation (also ohne Einschränkungen bezüglich des Rotationswinkels) um die Achse 13 rotiert werden kann, wahlweise auch in beiden Richtungen. Bei vielen bisherigen Röntgenbildgebungssystemen sind Einschränkungen bezüglich der Dynamik pro Rotationsgeschwindigkeit vorhanden, welche z.B. für jede Rotationsgeschwindigkeit eine eigene Kalibrierung notwendig machen. Mit der Einführung eines Schleifrings im Rotationsteil der Angulation kann der C-Bogen 11 uneingeschränkt rotieren und die Dynamik pro Rotationsgeschwindigkeit ist signifikant verbessert. Unterschiedliche konstante Rotationsgeschwindigkeiten sind problemlos möglich.

Das Röntgenbildgebungssystem weist außerdem eine Systemsteuerung 15 zur Ansteuerung von Systemfunktionen, z.B. Bewegungen des Aufnahmesystems oder die Applikation von Röntgenstrahlung. In diese integriert oder alternativ separat sind z.B. ein Bildbearbeitungssystem 20 zur Bearbeitung von Projektionsbildern und zur Rekonstruktion der Projektionsbilder zu Volumenbildern und eine Recheneinheit 16 vorhanden. Außerdem weist das Röntgenbildgebungssystem noch eine Speichereinheit 17, eine Eingabeeinheit 18, z.B. eine Tastatur oder einen Touchbildschirm, und eine Anzeigeeinheit 19, z.B. einen Monitor oder ein Touchbildschirm, auf.

In der FIG 1 ist ein Ablauf des erfindungsgemäßen Verfahrens gezeigt. In einem ersten Schritt 1 wird ein Kalibrierdatensatz, welcher Messdaten aus einer Vielzahl von Rotationen des Aufnahmesystems in Endlosrotation aufweist, bereitgestellt. Ein derartiger initialer Kalibrierdatensatz wird i.A. einmalig erstellt und kann dann für alle zukünftigen 3D- oder 4D-Aufnahmen verwendet werden. Der Kalibrierdatensatz sollte möglichst umfangreich erstellt werden und eine sehr große Anzahl von Kalibrierdaten enthalten, wobei eine sehr feine Abtastung der Bahn bei möglichst vielen verschiedenen Rotationsgeschwindigkeiten durchgeführt wird. Auf diese Weise können sehr viele unterschiedliche Stützstellen auf der Trajektorie zur Verfügung stehen. Der Kalibrierdatensatz wird i.A. in einer Speichereinheit oder einer Datenbank gespeichert und kann daraus bei Bedarf abgerufen und bereitgestellt werden.

In einem zweiten Schritt 2 (optional) werden ein oder mehrere Akquisitionsparameter aus der Gruppe Dauer der Akquisition, Anzahl der zu akquirierenden Projektionsbilder, aufzunehmender Winkelbereich der Akquisition für eine geplante 3D- oder 4D-Röntgenaufnahme mit dem Röntgenbildgebungssystem abgefragt. Dies kann z.B. über die Anzeigeeinheit 19 und die Eingabeeinheit 18 durchgeführt werden. Dem Nutzer kann also z.B. an der Anzeigeeinheit 19 ein oder mehrere Auswahlfenster angezeigt werden mit der Möglichkeit, dort eine freie Eingabe mittels der Eingabeeinheit durchzuführen. Macht der Nutzer die entsprechende(n) Eingabe(n) und gibt die entsprechenden Akquisitionsparameter ein, so werden in einem dritten Schritt 3 die eingegebenen Akquisitionsparameter von dem Röntgenbildgebungssystem, insbesondere der Systemsteuerung, entgegengenommen und zur Weiterverarbeitung verwendet, z.B. an die Recheneinheit 16 weitergegeben.

In einem vierten Schritt 4 wird anschließend unter Verwendung des Kalibrierdatensatzes und der entgegengenommenen Akquisitionsparameter ein individuelles Akquisitionsprotokoll berechnet, z.B. mittels der Recheneinheit. Das individuelle Akquisitionsprotokoll muss dabei nicht exakt so in dem Kalibrierdatensatz vorgegeben sein, sondern kann aufgrund der Tatsache, dass die Dynamik pro Rotationsgeschwindigkeit der Rotation des Aufnahmesystems so gleichmäßig ist, aus dem vorhandenen Kalibrierdatensatz flexibel berechnet werden. So kann z.B. die Dauer der Akquisition beliebig gewählt werden.

In einem fünften Schritt 5 wird anschließend das berechnete individuelle Akquisitionsprotokoll mittels der Systemsteuerung in das Röntgenbildgebungssystem geladen und in einem sechsten Schritt 6 wird dann die dem Akquisitionsprotokoll entsprechende 3D- oder 4D-Röntgenaufnahme durchgeführt. Anschließend können weitere Schritte, wie z.B. Anzeige von Bilddaten oder Bildverarbeitung und Rekonstruktion durchgeführt werden.

Durch das Verfahren und das erfindungsgemäße Röntgenbildgebungssystem wird eine Möglichkeit geschaffen, dass ein Nutzer Akquisitionsprotokolle für 3D- und 4D-Röntgenaufnahmen interaktiv, also jederzeit auch z.B. während einer Intervention patientenspezifisch definiert und unmittelbar die entsprechenden 3D- und 4D-Röntgenaufnahmen startet. Die Besonderheit liegt in der Umwandlung eines nur durch aufwändige Kalibrierung zu lösenden Dynamik-Problems mit Hilfe einer Vergrößerung des Angulationsbereichs (uneingeschränkt) in ein einfacheres Bahn-Sampling-Verfahren bei gleichbleibender kalibrierter Dynamik des C-Bogens pro Rotationsgeschwindigkeit. Es ist denkbar, mehrere Dynamiken (Rotationsgeschwindigkeiten) zu verwenden und jede für sich zu kalibrieren. Mit der Einführung eines Schleifrings im Rotationsteil der Angulation kann der C-Bogen uneingeschränkt rotieren. Unterschiedliche konstante Rotationsgeschwindigkeiten (und damit konstant bleibende Dynamik pro Rotationsgeschwindigkeit) sind denkbar.

Durch die Aufhebung der oberen Schranke des Rotationsbereichs kann die Dauer der Akquisition spezifiziert werden, indem etwa mit unterschiedlichem Abstand auf der Trajektorie Bilder für die 3D Rekonstruktion erfasst werden. Um dieses unterschiedliche Orts-Sampling zu ermöglichen, wird bei der initialen Kalibrierung die Bahn sehr fein abgetastet (pro Rotationsgeschwindigkeit), um unterschiedliche Stützstellen auf der Trajektorie bereitzustellen. Ferner ermöglicht dieses Konzept weitere Freiheitsgrade bzgl. der Definition von Akquisitionsprotokollen, welche während der Intervention durch den Benutzer spezifiziert werden können. Diese Freiheitsgrade umfassen bspw. die Anzahl der zu akquirierenden Projektionsbilder und den zu erfassenden Winkelbereich des Scans.

Die Erfindung lässt sich in folgender Weise kurz zusammenfassen: Für eine flexible Änderung des Akquisitionsprotokolls z.B. während interventioneller Eingriffe ist ein Verfahren zur Akquisition eines Bilddatensatzes mit einem Röntgenbildgebungssystem mit einem um ein Untersuchungsobjekt rotierbaren Aufnahmesystem, welches für eine Endlosrotation ausgebildet ist, mit den folgenden Schritten vorgesehen:
- Bereitstellung eines Kalibrierdatensatzes, welcher Messdaten aus einer Vielzahl von Rotationen des Aufnahmesystems in Endlosrotation aufweist,
- Entgegennahme zumindest eines auswählbaren Akquisitionsparameters, insbesondere aus der Gruppe Dauer der Akquisition, Anzahl der zu akquirierenden Projektionsbilder, aufzunehmender Winkelbereich der Akquisition und Winkelinkrement zwischen zwei aufeinander abfolgenden Projektionsbildern,
- Ermittlung, insbesondere Berechnung, eines Akquisitionsprotokolls mit dem oder den ausgewählten Akquisitionsparametern aus dem bereitgestellten Kalibrierdatensatz,
- Laden des ermittelten Akquisitionsprotokolls, und
- Aufnahme eines Bilddatensatzes unter Verwendung des ermittelten Akquisitionsprotokolls

## Patentansprüche

1. Verfahren zur Akquisition eines Bilddatensatzes mit einem Röntgenbildgebungssystem mit einem um ein Untersuchungsobjekt rotierbaren Aufnahmesystem, welches für eine Endlosrotation ausgebildet ist, mit den folgenden Schritten:
• Bereitstellung eines Kalibrierdatensatzes, welcher Messdaten aus einer Vielzahl von Rotationen des Aufnahmesystems in Endlosrotation aufweist,
• Entgegennahme zumindest eines auswählbaren Akquisitionsparameters,
• Ermittlung, insbesondere Berechnung, eines Akquisitionsprotokolls mit dem oder den ausgewählten Akquisitionsparametern aus dem bereitgestellten Kalibrierdatensatz,
• Laden des ermittelten Akquisitionsprotokolls, und
• Aufnahme eines Bilddatensatzes unter Verwendung des ermittelten Akquisitionsprotokolls.

2. Verfahren nach Anspruch 1, wobei zumindest ein auswählbarer Akquisitionsparameter aus der Gruppe: Dauer der Akquisition, Anzahl der zu akquirierenden Projektionsbilder, aufzunehmender Winkelbereich der Akquisition und Winkelinkrement zwischen je zwei aufeinander abfolgenden Projektionsbildern entgegengenommen wird.

3. Verfahren nach Anspruch 1 oder 2, wobei das Aufnahmesystem von einem C-Bogen gebildet wird, welcher einen Röntgendetektor und eine Röntgenquelle aufweist, und wobei der C-Bogen mittels einer Schleifringkonstruktion zur Durchführung der Endlosrotation aufgehängt ist.

4. Verfahren nach einem der vorangehenden Ansprüche, wobei zumindest ein weiterer Akquisitionsparameter aus der Gruppe Dauer der Akquisition, Anzahl der zu akquirierenden Projektionsbilder, aufzunehmender Winkelbereich der Akquisition und Winkelinkrement zwischen je zwei aufeinander abfolgenden Projektionsbildern entgegengenommen, ein weiteres Akquisitionsprotokoll berechnet, das weitere Akquisitionsprotokoll geladen und daraus ein weiterer Bilddatensatz aufgenommen wird.

5. Verfahren nach einem der vorangehenden Ansprüche, wobei eine Abfrage bezüglich des auswählbaren Akquisitionsparameters aus der Gruppe: Dauer der Akquisition, Anzahl der zu akquirierenden Projektionsbilder, aufzunehmender Winkelbereich der Akquisition und Winkelinkrement zwischen aufeinander abfolgenden Projektionsbildern, durchgeführt wird.

6. Verfahren nach einem der vorangehenden Ansprüche, wobei zumindest ein weiterer, über die Gruppe Dauer der Akquisition, Anzahl der zu akquirierenden Projektionsbilder, aufzunehmender Winkelbereich der Akquisition hinausgehender Akquisitionsparameter entgegen genommen und verwendet wird.

7. Röntgenbildgebungssystem zur Durchführung des Verfahrens nach einem der Ansprüche 1 bis 6 mit einem Aufnahmesystem mit einem Röntgendetektor und einer Röntgenquelle, die an einer Halterung angeordnet sind, welche Halterung dazu ausgebildet ist, in einer Endlosrotation um ein Untersuchungsobjekt zu rotieren, wobei das Röntgenbildgebungssystem dazu ausgebildet ist, während der Rotation des Aufnahmesystems eine Vielzahl von Projektionsbildern aus unterschiedlichen Projektionsrichtungen sowie einen Bilddatensatz gemäß einem Akquisitionsprotokoll aufzunehmen,
• aufweisend eine Systemsteuerung, welche dazu ausgebildet ist, einen zuvor aufgenommenen Kalibrierdatensatz, welcher Messdaten aus einer Vielzahl von Rotationen des Aufnahmesystems in Endlosrotation aufweist, bereitzustellen und ein Akquisitionsprotokoll zu laden,
• aufweisend eine Eingabeeinheit, welche zur Entgegennahme zumindest eines auswählbaren Akquisitionsparameters aus der Gruppe: Dauer der Akquisition, Anzahl der zu akquirierenden Projektionsbilder, aufzunehmender Winkelbereich der Akquisition und Winkelinkrement zwischen aufeinander abfolgenden Projektionsbildern, ausgebildet ist und
• aufweisend eine Recheneinheit, welche dazu ausgebildet ist, ein Akquisitionsprotokoll mit dem oder den ausgewählten Akquisitionsparametern aus dem bereitgestellten Kalibrierdatensatz zu berechnen.

8. Röntgenbildgebungssystem nach Anspruch 7, wobei die Halterung von einem C-Bogen gebildet wird und der C-Bogen mittels einer Schleifringkonstruktion aufgehängt ist, so dass eine Endlosrotation des C-Bogens ausführbar ist.

9. Röntgenbildgebungssystem nach einem der Ansprüche 7 oder 8, aufweisend eine Anzeigeeinheit zur Anzeige einer Abfrage bezüglich des oder der Akquisitionsparameter.

10. Röntgenbildgebungssystem nach einem der Ansprüche 7 bis 9, ausgebildet zur Entgegennahme und Verwendung zumindest eines weiteren, über die Gruppe Dauer der Akquisition, Anzahl der zu akquirierenden Projektionsbilder, aufzunehmender Winkelbereich der Akquisition und Winkelinkrement zwischen je zwei aufeinander abfolgenden Projektionsbildern hinausgehenden Akquisitionsparameters.

## Claims

1. Method for acquiring an image data record with an x-ray imaging system with a recording system which can be rotated around an examination object, which recording system is embodied for an endless rotation, with the following steps:
• Providing a calibration data record, which has measurement data from a plurality of rotations of the recording system in endless rotation,
• Receiving at least one selectable acquisition parameter,
• Determining, in particular calculating, an acquisition protocol with the selected acquisition parameter(s) from the provided calibration data record,
• Loading the determined acquisition protocol, and
• Recording an image data record using the determined acquisition protocol.

2. Method according to claim 1, wherein at least one selectable acquisition parameter is received from the group: period of acquisition, number of projection images to be acquired, angular region of the acquisition to be recorded and angular increment between every two sequential projection images.

3. Method according to claim 1 or 2, wherein the recording system is formed by a C-arm, which has an x-ray detector and an x-ray source, and wherein the C-arm is suspended by means of a slip ring structure in order to perform the endless rotation.

4. Method according to one of the preceding claims, wherein at least one further acquisition parameter is received from the group consisting of period of acquisition, number of projection images to be acquired, angular region of the acquisition to be recorded and angular increment between every two sequential projection images, a further acquisition protocol is calculated, the further acquisition protocol is loaded and a further image data record is recorded therefrom.

5. Method according to one of the preceding claims, wherein a query is performed regarding the selectable acquisition parameter from the group: period of acquisition, number of projection images to be acquired, angular region of the acquisition to be recorded and angular increment between sequential projection images.

6. Method according to one of the preceding claims, wherein at least one further acquisition parameter beyond the group consisting of period of acquisition, number of projection images to be acquired, angular region of the acquisition to be recorded is received and used.

7. X-ray imaging system for performing the method according to one of claims 1 to 6 with a recording system with an x-ray detector and an x-ray source, which are arranged on a bracket, which bracket is embodied to rotate in an endless rotation around an examination object, wherein the x-ray imaging system is embodied to record a plurality of projection images from different projection directions and an image data record in accordance with an acquisition protocol during the rotation of the recording system,
• having a system controller, which is embodied to provide a previously recorded calibration data record, which has measurement data from a plurality of rotations of the recording system in endless rotation, and to load an acquisition protocol,
• having an input unit, which is embodied for receiving at least one selectable acquisition parameter from the group: period of acquisition, number of projection images to be acquired, angular region of the acquisition to be recorded and angular increment between every two sequential projection images, and
• having a computing unit, which is embodied to calculate an acquisition protocol with the selected acquisition parameter(s) from the provided calibration data record.

8. X-ray imaging system according to claim 7, wherein the bracket is formed by a C-arm and the C-arm is suspended by means of a slip ring structure so that an endless rotation of the C-arm can be achieved.

9. X-ray imaging system according to one of claims 7 or 8, having a display unit for displaying a query relating to the acquisition parameter(s).

10. X-ray imaging system according to one of claims 7 to 9, embodied for receiving and using at least one further acquisition parameter beyond the group consisting of period of acquisition, number of projection images to be acquired, angular region of the acquisition to be recorded and angular increment between every two sequential projection images.

## Revendications

1. Procédé d'acquisition d'un jeu de données d'image par un système d'imagerie à rayons X, comprenant un système d'enregistrement pouvant tourner autour d'un objet à examiner, lequel est constitué pour une rotation sans fin, comprenant les stades suivants :
• on se procure un jeu de données d'étalonnage, qui a des données de mesure d'une pluralité de rotations du système d'enregistrement en rotation sans fin,
• on accepte au moins un paramètre d'acquisition pouvant être sélectionné,
• on détermine, notamment en calculant, un protocole d'acquisition ayant le ou les paramètres d'acquisition sélectionnés dans le jeu de données d'étalonnage mis à disposition,
• on charge le protocole d'acquisition déterminé et
• on enregistre un jeu de données d'images en utilisant le protocole d'acquisition déterminé.

2. Procédé suivant la revendication 1, dans lequel on accepte au moins un paramètre d'acquisition pouvant être sélectionné du groupe : durée d'acquisition, nombre des images de projection à acquérir, plage angulaire d'enregistrement de l'acquisition et incrément angulaire entre deux images de projection se succédant.

3. Procédé suivant la revendication 1 ou 2, dans lequel on forme le système d'enregistrement par un arceau en C, qui a un détecteur de rayons X et une source de rayons X, et dans lequel l'arceau en C est suspendu, au moyen d'une construction à bague collectrice, pour effectuer la rotation sans fin.

4. Procédé suivant l'une des revendications précédentes, dans lequel on accepte au moins un autre paramètre d'acquisition du groupe durée de l'acquisition, nombre des images de projection à acquérir, plage angulaire à enregistrer de l'acquisition et incrément angulaire entre deux images de projection se succédant, on calcule un autre protocole d'acquisition, on charge l'autre protocole d'acquisition et on en enregistre un autre jeu de données d'image.

5. Procédé suivant l'une des revendications précédentes, dans lequel on effectue une recherche en ce qui concerne le paramètre d'acquisition pouvant être sélectionné du groupe durée de l'acquisition, nombre des images de projection à acquérir, plage angulaire à enregistrer de l'acquisition et incrément angulaire entre deux images de projection se succédant.

6. Procédé suivant l'une des revendications précédentes, dans lequel on accepte et on utilise au moins un autre paramètre d'acquisition allant au-delà du groupe durée de l'acquisition, nombre des images de projection à acquérir, plage angulaire à enregistrer de l'acquisition entre deux images de projection se succédant.

7. Système d'imagerie à rayons X pour effectuer le procédé suivant l'une des revendications 1 à 6, comprenant un système d'enregistrement ayant un détecteur de rayons X et une source de rayons X, qui sont montés sur une fixation, laquelle fixation est constituée pour tourner suivant une rotation sans fin autour d'un objet à examiner, le système d'imagerie à rayons X étant constitué pour, pendant la rotation du système d'enregistrement, enregistrer une pluralité d'images de projection dans des directions de projection différentes, ainsi qu'un jeu de données d'images suivant un protocole d'acquisition,
• comportant une commande de système conçue pour mettre à disposition un jeu de données d'étalonnage enregistré auparavant, qui a des données de mesure d'une pluralité de rotations du système d'enregistrement en rotation sans fin et pour charger un protocole d'acquisition,
• comportant une unité d'entrée, qui est constituée pour accepter un paramètre d'acquisition pouvant être sélectionné du groupe durée de l'acquisition, nombre des images de projection à acquérir, plage angulaire à enregistrer de l'acquisition et incrément angulaire entre deux images de projection se succédant,
• comportant une unité de calcul, qui est constituée pour calculer un protocole d'acquisition ayant le ou les paramètres d'acquisition sélectionnés, à partir du jeu de données d'étalonnage mis à disposition.

8. Système d'imagerie à rayons X suivant la revendication 7, dans lequel la fixation est formée d'un arceau en C et l'arceau en C est suspendu au moyen d'une construction à bague collectrice, de manière à pouvoir exécuter une rotation sans fin de l'arceau en C.

9. Système d'imagerie à rayons X suivant l'une des revendications 7 ou 8, comportant une unité d'affichage pour afficher une interrogation concernant le ou les paramètres d'acquisition.

10. Système d'imagerie à rayons X suivant l'une des revendications 7 à 9, constitué pour accepter et utiliser au moins un autre paramètre d'acquisition allant au-delà du groupe durée de l'acquisition, nombre des images de projection à acquérir, plage angulaire à enregistrer de l'acquisition et incrément angulaire entre deux images de projection se succédant.
